(19) Europäisches Patentamt
European Patent Office
Office européen des brevets

(11) Veröffentlichungsnummer: **0 156 308**
A2

(12) # EUROPÄISCHE PATENTANMELDUNG

(21) Anmeldenummer: **85103301.9**

(22) Anmeldetag: **21.03.85**

(51) Int. Cl.⁴: **C 07 D 487/04**
G 03 G 5/06, C 07 D 231/38
//(C07D487/04, 249:00, 231:00)

(30) Priorität: **28.03.84 DE 3411387**

(43) Veröffentlichungstag der Anmeldung:
**02.10.85 Patentblatt 85/40**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Albert, Bernhard, Dr.**
**Eichenstrasse 60**
**D-6700 Ludwigshafen(DE)**

(72) Erfinder: **Hoffmann, Gerhard, Dr.**
**Pappelstrasse 22**
**D-6701 Otterstadt(DE)**

(72) Erfinder: **Neumann, Peter, Dr.**
**Franz-Schubert-Strasse 1**
**D-6908 Wiesloch(DE)**

(54) **Neue Pyrazolo3,4-dv-triazole und deren Verwendung.**

(57) Pyrazolo[3,4-d]v-triazole der Formel

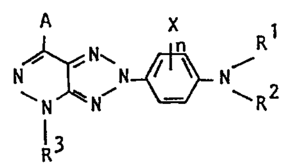

in der $R^1$, $R^2$ und $R^3$ für Alkyl, Cycloalkyl, gegebenenfalls substituiertes Phenyl oder Phenalkyl oder

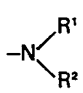

für einen gesättigten 5- oder 6-gliedrigen heterocyclischen Rest, X für Alkyl, Chlor, Brom, Fluor oder Phenyl, n für 0, 1 oder 2, A für Wasserstoff, Halogen,

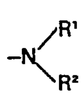,

Cyclohexylamino, Alkoxy, Alkoxyalkoxy, gegebenenfalls substituiertes Phenoxy, Phenalkoxy, Alkyl, Alkoxyalkyl, Phenalkyl oder gegebenenfalls substituiertes Phenyl stehen.

Die Verbindungen sind hervorragend als Ladungsträger transportierende Verbindungen in elektrophotographischen Aufzeichnungsmaterialien geeignet.

EP 0 156 308 A2

## Neue Pyrazolo[3,4-d]v-triazole und deren Verwendung

Die Erfindung betrifft neue Pyrazolo[3,4-d]v-triazole und deren Verwendung als Ladungsträger transportierende Verbindungen in elektrophotographischen Aufzeichnungsmaterialien.

Elektrophotographische Verfahren, dafür benötigte Materialien und verschiedene Varianten für den Aufbau von Aufzeichnungsmaterialien sind bekannt. Vorteilhaft für den Einsatz im Reproduktionssektor sind Materialien aus polymeren Bindemitteln, die an die speziellen Anforderungen des jeweiligen Einsatzgebietes angepaßt werden können, aus niedermolekularen organischen Verbindungen, die in den Bindemitteln auch in höheren Konzentrationen löslich und zu einem Transport von Ladungsträgern des elektrischen Stromes befähigt sind, sowie Verbindungen, insbesondere Farbstoffe oder Pigmente, die durch Absorption des bildmäßig eingestrahlten, aktinischen Lichtes Ladungsträger des elektrischen Stromes erzeugen und diese unter Mithilfe des von außen durch die elektrostatische Oberflächenladung aufgeprägten elektrischen Feldes auf die Ladung transportierenden Verbindungen übertragen können. Diese Ladungsträger erzeugenden Verbindungen können je nach Einsatzgebiet des Aufzeichnungsmaterials als eigene Schicht innerhalb einer Kompositstruktur eingebracht werden (DE-OS 22 20 408) oder in Form monodispers gelöster Farbstoffmoleküle in der Mischung aus Bindemittel und Ladungsträger transportierenden Verbindungen vorhanden sein (DE-PS 10 58 836). Das in der DE-OS 22 20 408 beschriebene mehrlagige elektrophotographische Aufzeichnungsmaterial besteht aus einem elektrisch leitfähigen Trägermaterial, einer ersten, Farbstoff enthaltenden, etwa 0,005 bis 2 µm dicken, durch Belichtung mit aktinischem Licht Ladungsträger des elektrischen Stromes erzeugenden Schicht und einer Schicht aus im Dunkeln isolierenden, organischen Materialien mit mindestens einer Ladungen transportierenden Verbindung.

Es ist auch bekannt, photohalbleitende organische Verbindungen zur Herstellung von elektrophotographischen Druckformen und insbesondere elektrophotographischen Offsetdruckformen zu verwenden (vgl. DE-PS 11 17 391 und 11 20 875, DE-AS 15 22 497 und 27 26 116).

Die gestiegenen Anforderungen an Reproduktionssysteme verlangen eine Vielzahl von Aufzeichnungsmaterialien und -systemen, um für spezielle Probleme optimale Lösungen aussuchen zu können. Gewünscht ist eine hohe Lichtempfindlichkeit, eine gute Auflösung und gute Betonerung. Die oft beanstandete ungenügende Betonerung, die auf eine ungünstige Feldstärkedifferenzierung zwischen belichteten und unbelichteten Flächen hinweist, ist hierbei oft auf eine zu hohe Dunkelleitfähigkeit des Aufzeichnungs-

Noe/Br

materials im beladenen Zustand zurückzuführen, so daß eine ungenügende Oberflächenladungsdichte vor der bildmäßigen Belichtung mit aktinischem Licht vorliegt.

Der vorliegenden Erfindung lag die Aufgabe zugrunde, neue Ladungsträger transportierende Verbindungen für elektrophotographische Aufzeichnungsmaterialien, insbesondere für die Herstellung von elektrophotographischen Druckformen wie Offsetdruckformen zu entwickeln, die bei hoher Lichtempfindlichkeit, guter Auflösung und einfacher Verarbeitung gleichzeitig ein geringes Dunkelleitvermögen aufweisen.

Es wurde nun gefunden, daß man verbesserte elektrophotographische Aufzeichnungsmaterialien mit elektrisch leitenden Trägern, Ladungsträgern erzeugenden Verbindungen bzw. Sensibilisatoren und Ladungsträger transportierenden Verbindungen erhält, wenn diese Materialien als Ladungsträger transportierende Verbindungen neue Pyrazolo[3,4-d]v-triazole der Formel (I)

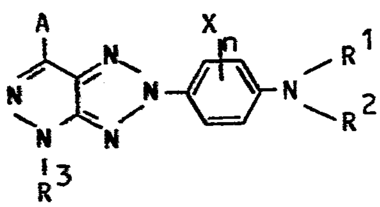 (I),

enthalten, in der

$R^1$, $R^2$ und $R^3$ unabhängig voneinander für $C_1$- bis $C_4$-Alkyl, $C_5$- bis $C_7$--Cycloalkyl, gegebenenfalls durch Chlor, Brom, $C_1$- bis $C_4$-Alkyl oder $C_1$- bis $C_4$-Alkoxy substituiertes Phenyl, Phenalkyl mit insgesamt 7 bis 10 C-Atomen oder

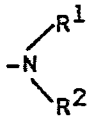 für einen gesättigten 5- oder 6-gliedrigen heterocyclischen Ring,

X für $C_1$- bis $C_4$-Alkyl, Chlor, Brom, Fluor oder Phenyl,

n für 0, 1 oder 2 und

A für 1) Wasserstoff, Halogen,

2) 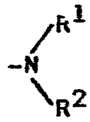 , worin $R^1$ und $R^2$ oder 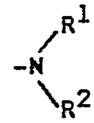 , die oben angegebene Bedeutung haben,

3) $C_1$- bis $C_6$-Alkoxy, $C_1$- bis $C_8$-Alkoxy-$C_2$- oder -$C_3$-alkoxy, gegebenenfalls durch Cl, Br, $C_1$- bis $C_4$-Alkyl substituiertes Phenoxy, Phenalkoxy mit insgesamt 7 bis 10 C-Atomen,

4) $C_1$- bis $C_6$-Alkyl, $C_1$- bis $C_8$-Alkoxy-$C_2$- oder -$C_3$-alkyl, gegebenenfalls durch Chlor, Brom oder $C_1$- bis $C_4$-Alkyl substituiertes Phenyl oder Phenalkyl mit insgesamt 7 bis 10 C-Atomen stehen.

Die erfindungsgemäßen elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind. Deutliche Vorteile weisen sie bei der Verwendung für die Herstellung von elektrophotographischen Druckformen auf und genügen hierbei hohen Ansprüchen im Hinblick auf das Auflösungsvermögen und die Druckauflage.

Als Substituenten $R^1$, $R^2$ und $R^3$ kommen für (I) z.B. im einzelnen in Betracht:

$C_1$- bis $C_4$-Alykl: Methyl, Ethyl, n- und i-Propyl, n-Butyl, Isobutyl;
$C_5$- bis $C_7$-Cycloalkyl: Cyclopentyl, Cyclohexyl, Cycloheptyl;
gegebenenfalls substituiertes Phenyl: Phenyl, 2-, 3- und 4-Tolyl, 2-, 3- und 4-Methoxyphenyl, 2-, 3- und 4-Ethoxyphenyl, 2-, 3- und 4-Chlorphenyl, 2-, 3- und 4-Bromphenyl, 3- und 4-Ethylphenyl, 3- und 4-Isopropylphenyl, 3- und 4-sec.-Butylphenyl;
Phenalkyl: Benzyl, 2- und 1-Ethylphenyl, 2- und 3-Propylphenyl, Butylphenyl.

Für 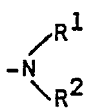 sind als gesättigte 5- und 6-gliedrige heterocyclische Ringe z.B. zu nennen: Reste des Pyrrolidins, Piperidins, Morpholins und N-$C_1$- bis $C_4$-Alkyl- und N-$C_2$- bis $C_4$-Hydroxyalkylpiperazins, wie die des N-Methyl-, N-Ethyl-, N-Butylpiperazins.

Für $R^1$, $R^2$ und $R^3$ sind $C_1$- bis $C_4$-Alkyl, insbesondere Methyl, Ethyl, Phenyl, und Benzyl, sowie für $R^3$ zusätzlich Cyclopentyl und für 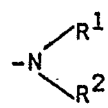 die Reste des Morpholins, Piperidins, Pyrrolidins und des N-$C_1$- bis $C_4$-Alkylpiperazins bevorzugt.

Als Substituenten X sind außer den bestimmt genannten im einzelnen z.B. zu nennen:
als $C_1$- bis $C_4$-Alkyl: Methyl, Ethyl, Propyl, Butyl.

Für X sind Chlor und Phenyl bevorzugt.

n ist 1 oder 2, vorzugsweise 0 (null).

Für A kommen im einzelnen in Betracht:

1) Wasserstoff; als Halogen Brom, Fluor, vorzugsweise Chlor;

2) $-N{<}^{R^1}_{R^2}$ , wobei $R^1$ und $R^2$ oder $-N{<}^{R^1}_{R^2}$ die oben angegebene Bedeutung haben, Cyclohexylamino;

3) $C_1$- bis $C_6$-Alkoxy: Methoxy, Ethoxy, n- und i-Propoxy, Butoxy, Pentoxy, Hexoxy;
$C_1$- bis $C_8$-Alkoxy-$C_2$- oder -$C_3$-alkoxy: 2-Methoxyethoxy, 2-Ethoxyethoxy, 2-Propoxyethoxy, 2-Butoxyethoxy, 2-Hexoxyethoxy, 2-Octoxyethoxy, 2-(2'-Ethylhexoxy)-ethoxy, 3-Methoxy-propoxy, 3-Ethoxypropoxy, 3-Propoxypropoxy, 3-Butoxypropoxy, 3-Hexoxypropoxy, 3-(2-Ethylhexoxy)-propoxy, 3-Octoxypropoxy;
gegebenenfalls substituiertes Phenoxy: Phenoxy, 3- und 4-Chlorphenoxy, 3- und 4-Bromphenoxy, 3- und 4-Methylphenoxy, 3- und 4-Ethylphenoxy;
Phenalkoxy: Benzyloxy, 2-Phenylethoxy, 2- und 3-Phenylpropoxy;

4) $C_1$- bis $C_4$-Alkyl: Methyl, Ethyl, n- und i-Propyl, Butyl;
$C_1$- bis $C_4$-Alkoxy-$C_2$- oder -$C_3$-Alkyl: 2-Methoxyethyl, 2-Ethoxyethyl, 2-Propoxyethyl, 2-Butoxyethyl, 3-Methoxypropyl, 3-Ethoxypropyl, 2-Propoxypropyl und 2-Butoxypropyl;
gegebenenfalls substituiertes Phenyl: Phenyl, 3- und 4-Chlorphenyl, 3- und 4-Bromphenyl, 3- und 4-Methylphenyl;
Phenalkyl: Benzyl, 2-Phenylethyl, 2- und 3-Phenylpropyl, 3- und 4-Butylphenyl.

Vorzugsweise stehen
$R^1$ und $R^2$ unabhängig voneinander für $C_1$- bis $C_4$-Alkyl, Phenyl oder Benzyl;
$R^3$ für $C_1$- bis $C_4$-Alkyl, Phenyl, Cyclohexyl oder Benzyl und
A für Wasserstoff, $C_1$- bis $C_4$-Alkyl, Phenyl oder Benzyl.

A steht insbesondere für Wasserstoff.

Aus anwendungstechnischen Gründen sind Pyrazolotriazole der Formel (II)

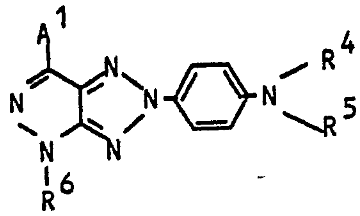

(II)

besonders bevorzugt, in der

$R^4$ und $R^5$ unabhängig voneinander für Methyl, Ethyl, Benzyl oder Phenyl,

$R^6$ für Methyl, Ethyl, Cyclohexyl, Benzyl oder Phenyl und

$A^1$ für Methyl, Ethyl, Benzyl, Phenyl oder insbesondere für Wasserstoff stehen.

Von den Verbindungen (II) sind aufgrund ihrer hervorragenden Eigenschaften die der Formel (III) besonders hervorzuheben:

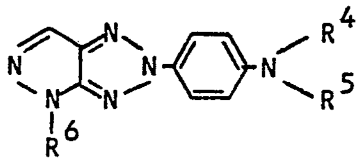

(III)

| | $R^4$ | $R^5$ | $R^6$ |
|---|---|---|---|
| III a) | $-C_2H_5$ | $-C_2H_5$ | $-CH_3$ |
| b) | $-C_2H_5$ | $-C_2H_5$ | $-C_2H_5$ |
| c) | $-C_2H_5$ | $-C_2H_5$ | $-CH_2-$ 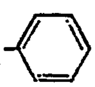 |
| d) | $-CH_3$ | $-CH_3$ | $-CH_2-$ 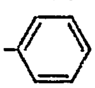 |
| e) | $-C_2H_5$ | $-C_2H_5$ | 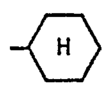 |
| f) | 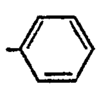 | 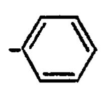 | $-C_2H_5$ |
| g) | $-CH_2-$ 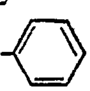 | $-CH_2-$ 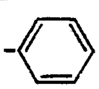 | $-CH_2-$ 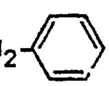 |
| h) | $-CH_3$ | 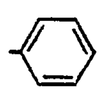 | $-C_2H_5$ |

Die neuen Pyrazolotriazole (I) können nach an sich bekannten Verfahren hergestellt werden.

So kann (I) auf folgendem Wege erhalten werden:

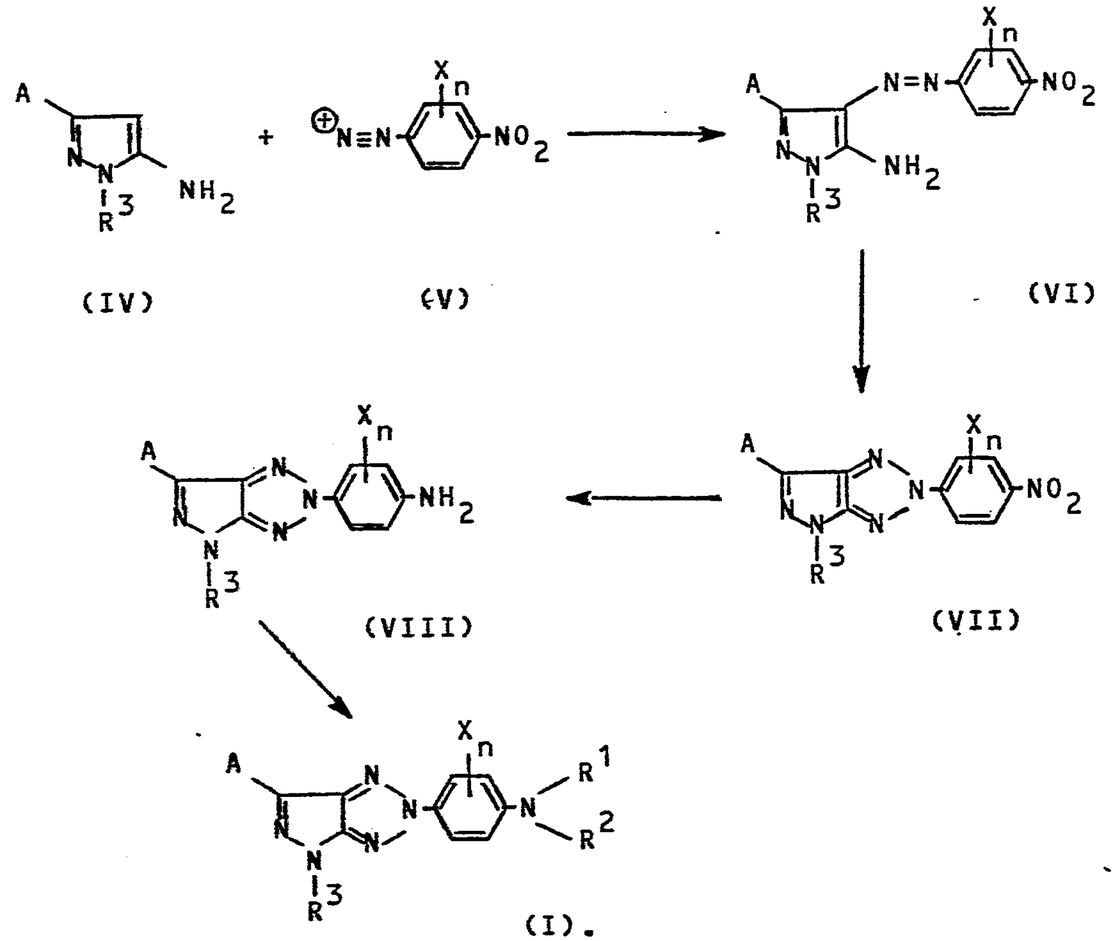

(IV) (V) (VI)

(VIII) (VII)

(I).

In den Formeln (I) bis (VIII) haben $R^1$, $R^2$, $R^3$, X, n und A die oben angegebene Bedeutung.

Der durch Kupplung von (V) auf (IV) erhaltene o-Aminoazofarbstoff (VI) wird oxidativ in an sich bekannter Weise in das Triazolderivat (VII) überführt.

Die Kupllung von (V) auf (IV) erfolgt in der Regel bei Temperaturen von -10 bis +20°C, vorzugsweise bei 0 bis 10°C.

Der Ringschluß von (VI) nach (VII) erfolgt durch Oxidation. Als Oxidationsmittel kommen hierfür z.B. in Betracht: Chromsäure, Dichromate, Hypochlorite, Wasserstoffperoxid, Blei-4-acetat, Kaliumhexacyanoferrat-III, Eisen-III-chlorid und Kupfer-II-sulfat. Chromsäure, die Chromate, Wasserstoffperoxid und Blei-4-acetat werden vorteilhafterweise in sauren Lösungsmitteln wie wäßrige Essigsäure oder Propionsäure, Hexacyanoferrat--III, vorteilhafterweise in basischen Lösungsmitteln wie Pyridin-Wasser--Gemischen angewendet.

Vorzugsweise erfolgt der Triazolringschluß durch Oxidation mit Kupfer-II--sulfat in einem Pyridin-Wasser-Gemisch, mit Hypochlorit in Tetrahydrofuran-Wasser oder mit Kupfer-II-salzen wie dem Sulfat oder dem Chlorid in

Methanol oder Methanol/Wasser in Gegenwart von Ammoniumsalzen, z.B. der der Mono- oder Dialkanolamine. Besonders bevorzugt ist der Triazolringschluß mit Hypochlorit in Tetrahydrofuran-Wasser-Gemischen.

Der Ringschluß erfolgt zweckmäßigerweise bei 20 bis 100°C, vorzugsweise bei 50 bis 70°C.

Die erhaltene p-Nitroverbindung (VII) kann nach den verschiedensten Methoden reduziert werden, z.B. katalytisch, mit Natriumborhydrid, Natriumdithionit, Natriumsulfit, Zink oder Eisen. Bevorzugt ist die katalytische Reduktion, die in Lösungsmitteln wie Essigsäureethylester, Ethanol, i-Propanol, Chloroform, Dimethylformamid oder N-Methylpyrrolidon erfolgen kann. Als Katalysator kann Raney-Nickel, Palladium oder Platin dienen.

Die Alkylierung der Aminogruppe in (VIII) kann mit den verschiedensten Reagentien nach an sich bekannten Verfahren erfolgen, z.B. mit Dialkylsulfaten, Alkylhalogeniden, Benzylhalogeniden, Alkyltosylaten oder Phenyljodiden in Lösungsmitteln wie Alkoholen, Aceton, Cyclohexanon, Tetrahydrofuran, Methoxyethanol, Ethoxyethanol, Dimethylformamid, N-Methylpyrrolidon in Gegenwart von basisch wirkenden Verbindungen wie Natriumhydrogencarbonat, Natriumcarbonat, Kaliumcarbonat, Natriumhydroxid, Kaliumhydroxid oder - in geeigneten Lösungsmitteln - auch Natriumhydrid.

Die Herstellung der Pyrazolotriazole der Formel (I) wird durch Ausführungsbeispiele erläutert.

Die neuen Pyrazolotriazole (I), (II) und (III) sind hervorragend als Ladungsträger transportierende Verbindungen in elektrophotographischen Schichten geeignet. Die neuen Verbindungen können mit Vorteil sowohl in einschichtigen als auch in mehrschichtigen auf elektroleitfähige Träger aufgebrachten Aufzeichnungssystemen verwendet werden. Für diesen Verwendungszweck haben sich Verbindungen der Formel (II) und insbesondere die der Formel (III) bewährt.

Geeignete einschichtige Systeme weisen bevorzugt auf einem leitfähigen Trägermaterial eine Schicht aus (a) 45 bis 75 Gewichtsteilen eines Bindemittels, (b) 30 bis 60, insbesondere 35 bis 50 Gewichtsteilen der erfindungsgemäßen Ladungsträger transportierenden Verbindungen I, (c) gegebenenfalls 5 bis 25 Gewichtsteilen eines weiteren, im wesentlichen inaktiven Bindemittels und (d) 0,05 bis 0,8 Gewichtsteilen einer bei aktinischer Belichtung Ladungsträger erzeugenden Verbindung, insbesondere eines geeigneten Farbstoffs auf. Die Schichten werden mit Vorteil aus einer ca. 5 gew.%igen Lösung in einem geeigneten organischen Lösungsmittel auf das

gereinigte leitfähige Trägermaterial so aufgebracht, daß nach dem Ablüften des Lösungsmittels je nach Verwendungszweck eine Trockenschichtdicke von ca. 0,8 bis 40 µm, bei elektrophotographischen Druckformen insbesondere 0,8 bis 6 µm, resultiert.

Geeignete Mehrschichtsysteme haben auf einem elektroleitfähigen Trägermaterial z.B. (a) eine Ladungsträger erzeugende Schicht und (b) eine Ladungstransportschicht aus 30 bis 60 Gewichtsteilen mindestens einer Ladungsträger transportierenden Verbindung der Formel I, 45 bis 75 Gewichtsteilen eines organischen Bindemittels und gegebenenfalls 5 bis 25 Gewichtsteilen weiterer, die mechanischen Eigenschaften der Schicht verbessernde Zusätze. Die erste Schicht wird vorteilhaft in einer Dicke von 0,005 bis 5 µm, insbesondere 0,1 bis 0,9 µm als Lösung in einem geeigneten Lösungsmittel auf das Trägermaterial aufgetragen. Nach dem Auftrag erfolgt der Auftrag der zweiten Schicht in einer Dicke, daß nach dem Trocknen der Kompositstruktur eine Schichtdicke von 5 bis 25, insbesondere 7 bis 15 µm resultiert.

Als elektrisch leitende Träger sind prinzipiell alle leitfähigen Trägermaterialien verwendbar, soweit sie für die vorgesehene Anwendung geeignet sind. Bevorzugt sind je nach dem Anwendungsgebiet der Aufzeichnungsmaterialien Aluminium-, Zink-, Magnesium-, Kupfer- oder Mehrmetallplatten, z.B. rohe oder vorbehandelte, z.B. aufgerauhte und/oder anodisierte Aluminiumbleche, Aluminiumfolien, Polymerfilme mit metallisierter Oberfläche wie aluminiumbedampfte Polyethylentetraphthalatfilme oder auch elektrisch leitende Spezialpapiere. Träger für Druckformen haben vorteilhaft eine Dicke von 0,08 bis ca. 0,3 mm.

Die Art der geeigneten organischen Bindemittel für die Schichten richtet sich nach dem beabsichtigten Verwendungszweck der Aufzeichnungsmaterialien. Für den Kopiersektor eignen sich z.B. Celluloseether, Polyesterharze, Polyvinylchloride, Polycarbonate, Copolymere, wie Styrol-Maleinsäureanhydrid-Copolymere oder Vinylchlorid-Maleinsäureanhydrid-Copolymere oder Mischungen solcher Bindemittel. Bei ihrer Auswahl spielen ihre filmbildenden und elektrischen Eigenschaften, ihre Haftfestigkeit auf dem Trägermaterial und ihre Löslichkeitseigenschaften eine besondere Rolle. Insbesondere bei Aufzeichnungsmaterialien für die Herstellung elektrophotographischer Druckplatten und besonders bei denen für den Offsetdruck sind solche besonders geeignet, die in basischen wäßrigen oder alkoholischen Lösungsmitteln löslich sind. Dies sind vor allem Substanzen mit alkalilöslich machenden Gruppen wie Anhydrid, Carboxyl-, Sulfonsäure-, Phenol- oder Sulfonimid-Gruppierungen. Bevorzugt sind Bindemittel, insbesondere solche mit hohen Säurenzahlen, die in basischen wäßrig-alkoholischen Lösungsmittelsystemen leicht löslich sind und ein mittleres Molekulargewicht (Gewichtsmittel) von 800 bis 80.000 und insbesondere 1.500 bis 50.000 aufweisen. Geeignet sind z.B. Copolymerisate aus Methacrylsäure und Methacrylsäureestern, besonders Copolymerisate aus Styrol und Maleinsäureanhydrid und aus Styrol, Methacrylsäure und Methacrylsäureester, soweit sie die vorstehende Löslichkeitsbedingung aufweisen. Obwohl bekanntermaßen Bindemittel mit freien Carboxylgruppen die Dunkelleitfähigkeit der elektrophotographischen Schichten in unerwünschter Weise erhöhen und dadurch zu schlechten Betonerungsergebnissen führen, lassen sich solche Bindemittel leicht an die erfindungsgemäß verwendeten Pyrazolotriazole anpassen. So hat sich gezeigt, daß Copolymerisate aus Styrol, Maleinsäureanhydrid und Acryl- oder Methacrylsäure, die einen Anteil von einpolymerisiertem Maleinsäureanhydrid von 5 bis 50 Gew.% und einen Anteil von einpolymerisierter Acryl- oder Methacrylsäure von 5 bis 35, insbesondere 10 bis 30 Gew.% aufweisen, befriedigende elektrophotographische Schichten mit hinreichender Dunkelleitfähigkeit ergeben. Sie weisen eine hervorragende Löslichkeit in Auswaschmitteln aus 75 Gew.% Wasser, 23 Gew.% Isobutanol und 2 Gew.% Soda auf, sind aber in offsettypischem Wischwasser unlöslich.

Geeignete Ladungsträger erzeugende Verbindungen bzw. Sensibilisatoren sind z.B. für einschichtig aufgetragene Systeme, wie sie auch zur Herstellung elektrophotographischer Druckformen dienen, Farbstoffe aus der Triarylmethanreihe, Xanthenfarbstoffe und Cyaninfarbstoffe. Sehr gute Ergebnisse wurden mit den erfindungsgemäßen Verbindungen der Formel I und Rhodamin B (C.I. 45170), Rhodamin 6 G (C.I. 45160), Malachitgrün (C.I. Basic Green 4; C.I. 4200), Methylviolett (C.I. 42535) oder Kristallviolett (C.I. 42555) erhalten. Bei mehrschichtig aufgetragenen Systemen liegt der Farbstoff oder das Pigment in einer separaten Ladungsträger erzeugenden Schicht vor. Hier sind Azofarbstoffe, Phthalocyanine, Isoindolinfarbstoffe und Perylentetracarbonsäurederivate gut wirksam. Besonders gute Ergebnisse werden mit Perylen-3,4:9,10-tetracarbonsäurediimidderivaten erzielt, wie sie in den DE-OS 31 10 954 und 31 10 960 beschrieben sind.

Für die jeweilige Verwendung kann das erfindungsgemäße elektrophotographische Aufzeichnungsmaterial übliche Zusätze enthalten, z.B. Verlaufmittel und Weichmacher in der photoleitfähigen Schicht oder Haftvermittler zwischen Träger und Schicht.

Die mit den erfindungsgemäßen Pyrazolotriazolen hergestellten elektrophotographischen Aufzeichnungsmaterialien zeichnen sich durch eine Kombination sehr guter Eigenschaften, insbesondere einer hohen Photoleitfähigkeit bei gleichzeitig sehr niedriger Dunkelleitfähigkeit aus, so daß die Schichten für die Kopiertechnik sehr geeignet sind.

Deutliche Vorteile weisen diese Materialien bei der Verwendung für die Herstellung von elektrophotographischen Druckformen auf und genügen hierbei hohen Ansprüchen im Hinblick auf das Auflösungsvermögen und die Druckauflage. Aus einer sehr randscharfen Bildwiedergabe resultiert eine gute Auflösung. Durch einen hohen Ladungskontrast können auch feine Rasterpunkte in den lichten Tonwortbereichen gut wiedergegeben werden. Ferner führt die Belichtung der Schichten zu sehr geringen Restspannungen und die bei der Betonerung erhaltenen Bilder zeichnen sich durch gute Grundfreiheit in den Nichtbildbereichen aus.

Die Herstellung elektrophotographischer Offsetdruckformen erfolgt dabei wie üblich durch eine elektrostatische Aufladung des elektrophotographischen Aufzeichnungsmaterials mittels einer Hochspannungscorona, eine direkt nachfolgende bildmäßige Belichtung, die Entwicklung des vorliegenden elektrostatischen, latenten Ladungsbildes mittels eines Trocken- oder Flüssigtoners, die Fixierung des Toners durch einen nachgeschalteten Schmelzvorgang und die Entfernung der unbetonerten photohalbleitenden Schicht mittels eines geeigneten Auswaschlösemittels. Die so erhaltene Druckform kann in bekannter Weise noch für den Offsetdruck vorbereitet werden, z.B. durch eine Hydrophilierung und Gummierung der wasserführenden Oberfläche.

Die Anwendung der Pyrazolotriazole der Formeln (I), (II) oder (III) wird in den Anwendungsbeispielen erläutert und belegt.

Die genannten Teile und Prozentangaben beziehen sich auf das Gewicht. NMP = N-Methylpyrrolidon, DMF = Dimethylformamid.

Beispiel 1

2-(4'-Dimethylaminophenyl)-4-benzyl-pyrazolo[3,4-d]v-triazol

a) Eine Mischung von 69 Teilen p-Nitroanilin, 125 Teilen konz. Salzsäure und 500 Teilen Wasser wird eine Stunde gerührt, dann werden 200 Teile Eis und anschließend eine Lösung von 34,5 Teilen Natriumnitrit in 100 Teilen Wasser zugegeben.

Nach einer Stunde gibt man 86,5 Teile 1-Benzyl-5-aminopyrazol in 500 Teilen Wasser und anschließend 81 Teile Natriumacetat dazu. Der Niederschlag wird abgesaugt und mit Wasser gewaschen.

Ausbeute: 163 Teile der Verbindung der Formel (VI) mit $R^3$ = Benzyl und X = A = H.
Schmelzpunkt: 212-215°C

b) 61 Teile der nach a) erhaltenen Verbindung, 200 Teile Tetrahydrofuran und 100 Teile Wasser werden bei 50°C unter Rühren mit 500 Teilen Chlorlauge versetzt und 6 Stunden bei dieser Temperatur gehalten. Nach dem Erkalten wird der Niederschlag abgesaugt und mit Wasser gewaschen.

Ausbeute: 70 Teile der Verbindung der Formel (VII) mit $R^3$ = Benzyl und X=A=H.
Schmelzpunkt: 178-181°C.

c) 20 Teile der nach b) erhaltenen Verbindung werden in 200 Teilen DMF in Gegenwart von Raney-Nickel bei 60°C hydriert. Nach dem Erkalten wird die Reaktionsmischung vom Katalysator abfiltriert und in Wasser gegossen. Der Niederschlag wird abfiltriert und mit Wasser gewaschen.

Ausbeute: 9,5 Teile der Verbindung der Formel (VIII) mit $R^3$ = Benzyl und X=A=H.
Schmelzpunkt: 106-108°C.

d) 22 Teile des nach c) erhaltenen Amins werden in einem Gemisch aus 300 Teilen Dimethylformamid, 20 Teilen Formaldehydlösung (30 %ig) und 20 Teilen Ameisensäure 12 Stunden auf 100°C erhitzt. Das Reaktionsgemisch wird im Vakuum eingeengt und der Rückstand aus Alkohol umkristallisiert.

Ausbeute: 22 Teile der Verbindung der Formel (IIId),
Schmelzpunkt: 173-174°C.

Beispiel 2

10,2 Teile der nach Beispiel 1c) erhaltenen Aminoverbindung werden in 50 Teilen NMP mit 9,8 Teilen Bromethan und 8,0 Teilen $NaHCO_3$ 7 Stunden bei 100°C gerührt. Nach dem Abkühlen wurde mit Wasser gefällt, die Fällung abgesaugt und mit Wasser gewaschen.

Ausbeute: 5,3 Teile der Verbindung (IIIc).
Schmelzpunkt: 125-126°C.

Beispiele 3 bis 16

Analog Beispiel 1 oder 2 wurden Pyrazolotriazole der Formel

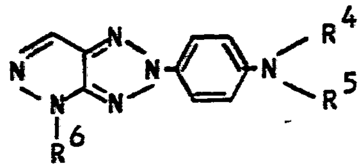

(III)

hergestellt.

Die Bedeutung von $R^4$, $R^5$ und $R^6$ ist in der folgenden Tabelle angegeben.

| Bsp. | $R^4$ | $R^5$ | $R^6$ | Schmp. [°C] |
|---|---|---|---|---|
| 3 | $-C_2H_5$ | $-C_2H_5$ | $-CH_3$ | 138-140 |
| 4 | $-C_2H_5$ | $-C_2H_5$ | $-C_2H_5$ | 114-116 |
| 5 | $-C_2H_5$ | $-C_2H_5$ | $-CH_2-$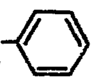 | 125-126 |
| 6 | $-CH_3$ | $-CH_3$ | $-CH_2-$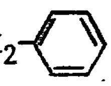 | 173-174 |
| 7 | $-C_2H_5$ | $-C_2H_5$ | 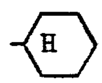 | 133-135 |
| 8 | 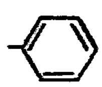 | 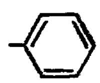 | $-C_2H_5$ | 152-154 |
| 9 | $-CH_2-$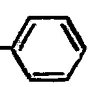 | $-CH_2-$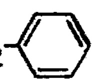 | $-CH_2-$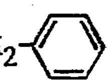 | 188-190 |
| 10 | $-CH_3$ | 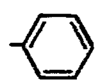 | $-C_2H_5$ | 164-166 |
| 11 | $-CH_3$ | $-CH_3$ | $-C_2H_5$ | 189-191 |
| 12 | $-CH_2-$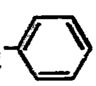 | $-CH_2-$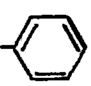 | $-C_2H_5$ | 147-149 |
| 13 | 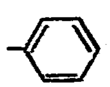 | 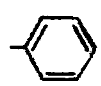 | $-CH_2-$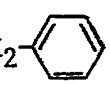 | 173-175 |
| 14 | $-C_2H_5$ | $-C_2H_5$ | 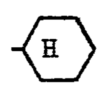 | 111-113 |
| 15 | $-CH_3$ | $-CH_3$ | 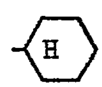 | 123-124 |
| 16 | $-CH_2-$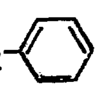 | $-CH_2-$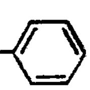 | 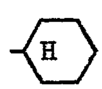 | 135-137 |

Anwendungsbeispiel 1

Auf eine Polyethylenterephthalatfolie mit einer aufgedampften, leitfähigen Aluminiumschicht in einer Dicke von etwa 300 Å wird eine Schicht aus 60 Teilen eines chlorierten Perylen-3,4:9,10-tetracarbonsäurediimidbis-benzimidazols mit einem Chlorgehalt von etwa 38 % und 50 Teilen eines Copolymerisates aus Vinylchlorid, Acrylsäure und einem Maleinsäurediester in einer Dicke von etwa 0,85 µm als Ladungsträger erzeugende Schicht aufgebracht.

Auf diese Ladungsträger erzeugende Schicht wird mit Hilfe einer Lösung aus 55 Teilen eines handelsüblichen Bindemittels auf der Basis Polycarbonat mit einem Schmelzbereich von 220 bis 230°C und 40 Teilen der Verbindung aus Beispiel 5 in Essigsäureethylester so aufgebracht, daß nach dem Ablüften und Trocknen (30 min bei 80°C) eine Schichtdicke von 12 µm vorhanden ist.

Das so erhaltene Aufzeichnungsmaterial besitzt eine Halbwertsfotoempfindlichkeit von ca. 0,6 $J.m^2$.

Die Schicht zeigt im Dunkeln innerhalb von etwa 0,4 Sekunden einen Potentialabfall von 600 auf 598 Volt, während in der gleichen Schicht in der gleichen Zeit bei Belichtung mit einer Beleuchtungsstärke von 0,60 $mW.cm^{-2}$ das Potential von 600 auf 300 Volt abfällt. Die maximale Beladbarkeit ist mit größer 1300 Volt weit über den in Kopiergeräten erforderlichen Oberflächenpotentialen (ca. 700 Volt), so daß die Schicht für die Kopiertechnik sehr geeignet ist.

Anwendungsbeispiel 2

50 Teile eines Copolymerisats aus 70 % Styrol, 24 % Acrylsäure und 6 % Maleinsäureanhydrid mit einem mittleren Molekulargewicht $M_w$ von 10.000, 50 Teile des Pyrazolotriazols aus Beispiel 5 und 0,5 Teile C.I. Basic Violet; J.I.No. 45170 werden aus einer 5 %igen Lösung in Tetrahydrofuran auf eine elektrolytisch aufgerauhte und anodisierte Aluminiumfolie von 0,15 mm Dicke in einer Trockenschichtdicke von etwa 4 µm aufgebracht.

Diese Druckplatte wird nach einer Aufladung mittels einer Hochspannungscorona auf ein Oberflächenpotential von etwa -600 V in einer Kamera bildmäßig 85 Sekunden belichtet. Danach wird mit einem Pulvertoner entwickelt, der bei 160°C abriebfest eingebrannt wird. Die unbetonerte Schicht wird mittels einem Gemisch aus 5 % Soda, 25 % Isopropanol und 74,5 % Wasser abgewaschen, wodurch die Aluminiumoberfläche freigelegt

wird. (Die Lösungen werden mit einem Wattebausch über die Schicht gestrichen.) Man erhält die im Offsetdruck erwünschte Differenzierung in hydrophile und oleophile Bereiche, wobei die Trägeroberfläche die hydrophilen Bereiche liefert.

Anschließend an die Behandlung mit der alkalischen Flüssigkeit wird die Druckplatte mit Wasser nachgespült und durch Überwischen mit verdünnter Phosphorsäurelösung die Hydrophilie der Trägeroberfläche weiter erhöht. Nach Einfärben mit fetter Farbe kann mit den Platten in bekannter Weise auf Offsetdruckmaschinen damit gedruckt werden.

Patentansprüche

1. Neue Pyrazolo[3,4-d]v-triazole der Formel

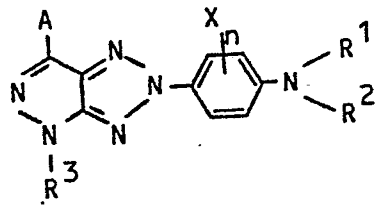

in der $R^1$, $R^2$ und $R^3$ unabhängig voneinander für $C_1$- bis $C_4$-Alkyl, $C_5$- bis $C_7$-Cycloalkyl, gegebenenfalls durch Chlor, Brom, $C_1$- bis $C_4$-Alkyl oder $C_1$- bis $C_4$-Alkoxy substituiertes Phenyl oder Phenalkyl mit insgesamt 7 bis 10 C-Atomen oder

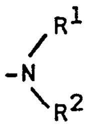 für einen gesättigten 5- oder 6-gliedrigen heterocyclischen Ring,

X für $C_1$- bis $C_4$-Alkyl, Chlor, Brom, Fluor oder Phenyl,

n für 0, 1 oder 2 und

A für 1) Wasserstoff, Halogen,

2) 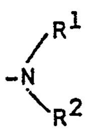 , worin $R^1$ und $R^2$ oder 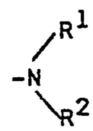 die oben angegebene Bedeutung haben, oder Cyclohexylamino;

3) $C_1$- bis $C_6$-Alkoxy, $C_1$- bis $C_8$-Alkoxy-$C_2$- oder -$C_3$-alkoxy, gegebenenfalls durch Chlor, Brom, $C_1$- bis $C_4$-Alkyl substituiertes Phenoxy, Phenalkoxy mit insgesamt 7 bis 10 C-Atomen, 4) $C_1$- bis $C_6$-Alkyl, $C_1$- bis $C_8$-Alkoxy-$C_2$- oder -$C_3$-alkyl, Phenalkyl mit insgesamt 7 bis 10 C-Atomen oder gegebenenfalls durch Chlor, Brom oder $C_1$- bis $C_4$--Alkyl substituiertes Phenyl stehen.

2. Pyrazolotriazole gemäß Anspruch 1, dadurch gekennzeichnet, daß n = 0 (null) ist.

3. Pyrazolotriazole gemäß Anspruch 1 oder 2, dadurch gekennzeichnet, daß $R^1$ und $R^2$ unabhängig voneinander für $C_1$- bis $C_4$-Alkyl, Phenyl oder Benzyl, $R^3$ für $C_1$- bis $C_4$-Alkyl, Phenyl, Cyclohexyl oder Benzyl und A für Wasserstoff, $C_1$- bis $C_4$-Alkyl, Phenyl oder Benzyl stehen.

4. Pyrazolotriazole gemäß Anspruch 1, 2 oder 3, <u>dadurch gekennzeichnet</u>, daß A für Wasserstoff steht.

5. Pyrazolotriazole gemäß Anspruch 1, <u>gekennzeichnet durch</u> die Formel

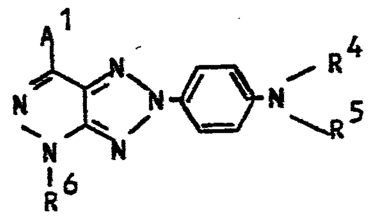

in der $R^4$ und $R^5$ unabhängig voneinander für Methyl, Ethyl, Benzyl oder Phenyl, $R^6$ für Methyl, Ethyl, Cyclohexyl, Benzyl oder Phenyl und $A^1$ für Wasserstoff, Methyl, Ethyl, Benzyl oder Phenyl stehen.

6. Pyrazolotriazole gemäß Anspruch 5, <u>dadurch gekennzeichnet</u>, daß $A^1$ für Wasserstoff steht.

7. Pyrazolotriazole gemäß Anspruch 6, <u>dadurch gekennzeichnet</u>, daß

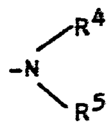 für Dimethylamino, Diethylamino, Dibenzylamino, N-Methyl-N--phenylamino oder Diphenylamino und $R^6$ für Methyl, Ethyl, Cyclohexyl oder Benzyl stehen.

8. Pyrazolotriazol der Formel

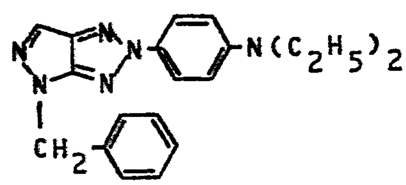 .

9. Verwendung der Pyrazolotriazole gemäß den Ansprüchen 1 bis 8 als Ladungsträger transportierende Verbindungen in elektrophotographischen Aufzeichnungsmaterialien.